# EUROPÄISCHE PATENTSCHRIFT

(11) **EP 0 264 702 B1**
(45) Veröffentlichungstag und Bekanntmachung des Hinweises auf die Patenterteilung: **02.08.1995**
(21) Anmeldenummer: 87114510.8
(22) Anmeldetag: 05.10.1987
(51) Int. Cl.: C08K 5/12, C07C 69/76

(54) **Kunststoff, der einen Tetracarbonsäuretetraester-Weichmacher enthält**
Synthetic polymer containing a plastifying tetraester of a tetracarboxylic acid
Polymère synthétique contenant un plastifiant tétraester d'acide tétracarboxylique

(30) Priorität: 07.10.1986 CH 4007/86
(43) Veröffentlichungstag der Anmeldung: 27.04.1988
(73) Patentinhaber: Willi Möller AG, CH-8050 Zürich (CH)
(72) Erfinder: Simon, Wilhelm, Prof. Dr. Dipl.-Ing. Chem. ETH, CH-8006 Zürich (CH); Oesch, Urs, Dr., MA. 01746 (US)
(74) Vertreter: Blum, Rudolf Emil Ernst

(56) Entgegenhaltungen:
- EP-A- 219 742
- JP-A- 5 974 144
- US-A- 3 293 267
- US-A- 3 332 964

## Beschreibung

### HINTERGRUND DER ERFINDUNG

Die vorliegende Erfindung betrifft Kunststoffe, die einen Tetracarbonsäuretetraester-Weichmacher enthalten. Der Weichmacher ist ein Benzhydrol-tetracarbonsäuretetraester, in dem die veresternden Alkoholkomponenten Alkanole, Alkenole oder Alkinole mit 4 - 24 Kohlenstoffatomen sind.

Dieser Tetraester-Weichmacher ist in der Kunststoffmatrix gut verankert, so dass bei langer Lagerung des Kunststoffes keine Wanderung des Weichmachers in dem Kunststoffgrundgerüst auftritt. Dadurch wandert der Tetracarbonsäuretetraester bei Kontakt des Kunststoffes mit anderen Materialien nicht in diese ein und der Tetracarbonsäuretetraester-Weichmacher wird auch bei Kontakt des Kunststoffes mit flüssigen Medien nicht ausgewaschen.

Der Kunststoff enthält zusätzlich zu dem Tetracarbonsäuretetraester-Weichmacher noch eine ionenempfindliche Komponente. Auch diese Komponente wird durch den Tetracarbonsäuretetraester-Weichmacher in der Kunststoffmatrix verankert. Entsprechende ionenselektive Teile zeichnen sich dementsprechend durch eine besonders lange Lebensdauer aus.

Werden besonders hohe Mengen an dem Tetracarbonsäuretetraester-Weichmacher eingesetzt, beispielsweise pro 10 Gew.-Teile des Kunststoffes, 12 - 90 Gew.-Teile des Benzhydrol-tetracarbonsäuretetraesters, dann erhält man Kunststoffe mit hoher Elastizität und sehr guter Haftfähigkeit.

### BESCHREIBUNG DES STANDES DER TECHNIK

Es ist allgemein bekannt, Weichmacher einzusetzen, um spröde Kunststoffe oder zu wenig biegbare Kunststoffe für verschiedene Einsatzgebiete geeignet zu machen.

In der USA-Patentschrift Nr. 3 389 168 von J.W. Hirzy werden Diallyl-benzophenon-tetra-carbonsäureester beschrieben, welche die folgende Formel 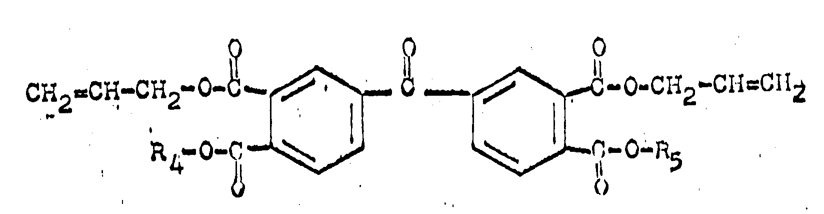
aufweisen, in welcher
R₄ und R₅ Allylgruppen, Alkylgruppen mit 1 - 18 Kohlenstoffatomen oder Cycloalkylgruppen mit 6 - 18 Kohlenstoffatomen sind. Diese Benzophenon-tetracarbonsäuretetraester, in denen mindestens zwei der Ester bildenden Alkoholgruppen nur drei Kohlenstoffatome aufweisen, nämlich die beiden Allylgruppen, werden als Weichmacher und Vernetzungsmittel für Halogen enthaltende Polymere eingesetzt. Gemäss den Beispielen 16 und 17 dieses Patentes werden pro 100 Gew.-Teile an Polyvinylchlorid 60 Gew.-Teile dieser Weichmacher verwendet.

In der japanischen Patentveröffentlichung Nr. 59-74,144 von Kokai Tokkyo Koho und in dem entsprechenden japanischen Patentabstracts, Band 8, Nr. 178 (C-338) (1616), 6. August 1984, werden Weichmacher für Vinylchloridpolymere beschrieben, welche die folgende Formel A 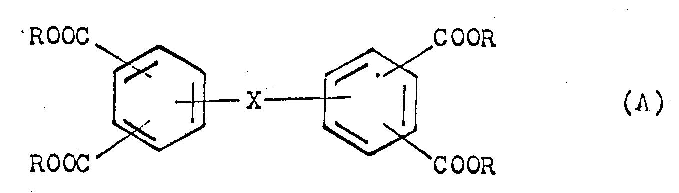
aufweisen, worin R ein gesättigter aliphatischer Kohlenwasserstoffrest mit 4 - 18 Kohlenstoffatomen ist und
X ein Sauerstoffatom, ein Schwefelatom, eine Gruppe der Formel 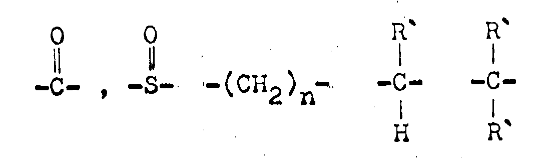
worin n 1 - 6 ist und R 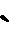 für eine Methylgruppe oder eine Trifluormethylgruppe steht, bedeutet oder X die Bedeutung bestimmter zweiwertiger organischer Reste besitzt, die über je eine Aethergruppierung an die beiden Benzolkerne gebunden sind. Pro 100 Gew.-Teile des Vinylchloridkunststoffes müssen in diesem Falle 20 - 120 Gew.-Teile, vorzugsweise 30 - 100 Gew.-Teile, des Tetracarbonsäuretetraesters der Formel A verwendet werden.

Wenn in der Formel A X eine Carbonylgruppe ist, dann sind diese Verbindungen Benzophenon-tetracarbonsäure-tetraester. Die mit den Verbindungen der Formel A weichgemachten Polyvinylchloride Zeigten bei den dort angewandten Weichmachermengen, welche pro 10 Gew.-Teilen an Kunststoff 2 - 12 Gew.-Teile dieses Weichmachers betrugen, ein nur sehr geringes Ausbluten und Abdampfen des Weichmachers aus der Weichmacher enthaltenden Vinylchloridzusammensetzung.

In der nicht zum Stande der Technik gehörenden europäischen Patentveröffentlichung 0 219 742 des Anmelders sind Kunststoff-Formkörper mit hoher Elastizität und Haftfähigkeit beschrieben, die als plastifizierende Komponente sehr hohe Anteile an einem Benzophenon-Tetracarbonsäure-tetraester enthalten, nämlich pro 10 Gew.-Teile des Kunststoffes, 7 bis 90 Gew.-Teile an dem Benzophenon-Tetracarbonsäure-tetraester.

In der USA Patentschrift Nr. 3 293 267 sind Verbindungen der folgenden Formel B 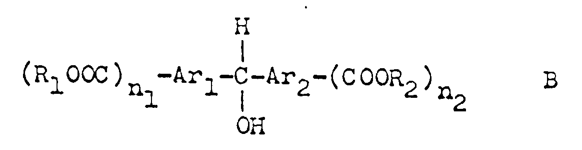
beschrieben, in welchen die Reste
Ar₁ und Ar₂ aromatische Reste oder heteroaromatische Reste sind, beispielsweise Phenylreste, Biphenylreste oder Naphthylreste und
R₁ und R₂ Alkylreste mit 1 - 19 Kohlenstoffatomen bedeuten, und
n₁ und n₂ jeweils den Wert 1 oder 2 aufweisen.

Wenn in diesen Verbindungen n₁ und n₂ 2 sind und Ar₁ und Ar₂ Phenyl bedeuten, dann sind diese Tetracarbonsäure-tetraester die entsprechenden Ester der Benzhydrol-tetracarbonsäure. In Spalte 1, Zeilen 21 - 24 der entsprechenden Patentschrift wird ferner erwähnt, dass die fraglichen Ester als Zwischenprodukt bei der Herstellung von Weichmachern, Kunststoffen und Fasern eingesetzt werden können. Der fraglichen Patentschrift ist jedoch kein Hinweis zu entnehmen, dass die entsprechenden Ester selber als Weichmacher wirken.

In der USA Patentschrift 3,332,964 sind ferner Tetracarbonsäure-tetraester beschrieben, bei denen die vier Estergruppierungen an die aromatischen Kerne von Diphenylmethan gebunden sind. Auch in dieser Patentschrift wird erwähnt, dass die fraglichen Produkte als Zwischenprodukt bei der Herstellung von Weichmachern eingesetzt werden können. Es ist jedoch dieser Patentschrift kein Hinweis zu entnehmen, dass die entsprechenden Tetracarbonsäure-tetraester selbst als Weichmacher wirken.

Es ist schliesslich bekannt, in ionenselektiven Membranen zur Bestimmung der Konzentration von Ionen eine ionenselektive Komponente für das zu bestimmende Kation oder Anion in einer Kunststoffmatrix einzusetzen. Derartige ionenselektive Membranen enthalten im allgemeinen ausserdem einen Weichmacher, der unter anderem dazu dient, die Elektrodenstabilität von Elektroden, die als ionenempfindlichen Teil derartige Membranen besitzen, zu erhöhen und die Empfindlichkeit der Membranen zu steigern. Typische Beispiele für Weichmacher, die bisher in ionenempfindlichen Membranen eingesetzt wurden, sind die Ester von Dicarbonsäuren, wie zum Beispiel Ester der Phthalsäure, der Sebacinsäure oder der Adipinsäure, sowie ferner aromatische Aether, aliphatische Aether und Ester der Phosphorsäure, wie zum Beispiel gemischte aromatische und aliphatische Phosphorsäureester.

Es wurde jetzt überraschenderweise gefunden, dass Benzhydrol-tetracarbonsäure-tetraester, in welchen die veresternde Alkoholkomponente 4 - 24 Kohlenstoffatome aufweist, hervorragende Eigenschaften besitzen, wenn sie als Weichmacher in Kunststoffen eingesetzt werden. Diese Tetracarbonsäure-tetraester-Weichmacher umfassen also auch diejenigen Verbindungen, die in der USA-Patentschrift 3,293,267 beschrieben sind.

Wenn die entsprechenden Tetracarbonsäure-tetraester als Weichmacher in Kunststoffen eingesetzt werden, dann sind sie fest in der Matrix des Kunststoffes verankert und es wird so auch bei langer Lagerung eine Wanderung in dem Kunststoff, eine Anreicherung an der Oberfläche des Kunststoffes und ein Uebertreten dieser Tetraester-Weichmacher in feste Stoffe oder Flüssigkeiten, die mit dem weichgemachten Kunststoff in Berührung stehen, verhindert. Ueberraschenderweise zeigte es sich (siehe Ausführungsbeispiele) dass auch bei sehr hohen Gehalten an dem Benzhydroltetracarbonsäure-tetraester-Weichmacher in dem Kunststoff, nämlich bis zu 90 Gew.-Teilen Weichmacher pro 10 Gew.-Teilen Kunststoff, eine feste Verankerung des Weichmachers in der Kunststoffmatrix erfolgt.

### BESCHREIBUNG DER ERFINDUNG

Gegenstand der vorliegenden Erfindung ist ein Kunststoff, der einen Benzhydrol-tetracarbonsäuretetraester-Weichmacher enthält, wobei der Weichmacher in der Matrix des Kunststoffes verankert ist, wodurch eine Wanderung des Weichmachers in dem Kunststoff bei langen Lagerungszeiten verhindert wird und eine Auslaugung des Weichmachers aus dem Kunststoff bei Berührung des Kunststoffes mit festen und flüssigen Materialien verhindert wird. In dem Benzhydrol-tetracarbonsäure-tetraester sind die Carboxylatgruppen des Tetraesters direkt an die aromatischen Kerne des Benzhydroles gebunden und die veresternden Alkoholkomponenten dieses Tetraester-Weichmachers sind Alkanole, Alkenole oder Alkinole mit 4 - 24 Kohlenstoffatomen, wobei gegebenenfalls in den aromatischen Ringen des Benzhydrol-Grundgerüstes und/oder in den Resten der Alkoholkomponente nicht-ionische Substituenten vorhanden sind.

Als weiteren Bestandteil enthält der Kunststoff eine ionenselektive Komponente.

Diese Kunststoffe besitzen eine hohe Elastizität und Haftfähigkeit, wenn sie pro 10 Gew.-Teile des Kunststoffes 12 - 90 Gew.-Teile, vorzugsweise 15 - 80 Gew.-Teile, und speziell bevorzugt 20 - 70 Gew.-Teile dieses Weichmachers enthalter, wobei der Weichmacher in der Matrix des Kunststoffes verankert ist, wodurch eine Wanderung des Weichmachers in dem Kunststoff bei langen Lagerungszeiten verhindert wird und eine Auslaugung des Weichmachers aus dem Kunststoff bei Berührung des Kunststoffes mit festen und flüssigen Materialien verhindert wird.

Zu den Benzhydrol-tetracarbonsäure-tetraestern, die als Weichmacher in den erfindungsgemässen Kunststoffzusammensetzungen eingesetzt werden, gehören auch solche Tetracarbonsäure-tetraester, der weiter vorne angegebenen Formel B, die in der USA-Patentschrift 3,293,267 bereits beschrieben sind.

Bevorzugte Benzhydrol-tetracarbonsäuretetraester, die in den erfindungsgemässen Kunststoffzusammensetzungen als Weichmacher eingesetzt werden, sind diejenigen, in denen die Alkoholkomponente des Tetraesters ein geradkettiger Alkohol oder ein höchstens drei Verzweigungsstellen aufweisender Alkohol ist.

In den Benznydrol-tetracarbonsäuretetraestern trägt vorzugsweise jeder der beiden Benzolringe zwei Estergruppierungen. Speziell bevorzugt ist es dabei, wenn in den beiden Benzolkernen die Anordnung der Estergruppierungen, bezogen auf das Kohlenstoffatom der Benzolkerne, welches an die Gruppierung

〉CH-OH

des Benzhydroles gebunden ist, identisch ist.

Speziell bevorzugte Benzhydrol-tetracarbonsäuretetraester weisen die folgende Formel I 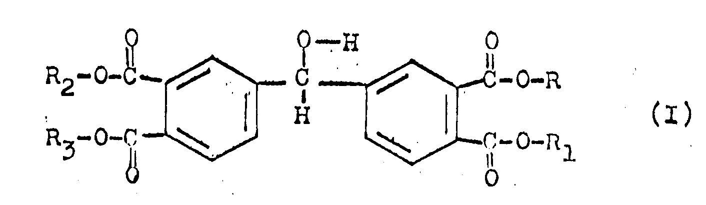
auf, wobei Jeder der Reste R, R₁, R₂ und R₃ ein Alkylrest, Alkenylrest oder Alkinylrest mit 4 bis 22 Kohlenstoffatomen, Vorzugsweise 6 bis 20 Kohlenstoffatomen, ist und wobei Vorzugsweise die Reste R, R₁, R₂, bzw. R₃ geradkettig sind oder höchstens eine Verzweigungsstelle aufweisen.

Aufgrund der Symmetrie des Molekülaufbaues und auch wegen der leichteren Herstellbarkeit sind solche Benzhydrol-tetracarbonsäuretetraester bevorzugt, in denen die vier Estergruppierungen die gleiche Struktur besitzen. Bei den bevorzugten Benzhydrol-tetracarbonsäuretetraestern der oben angegebenen Formel I bedeutet dies, dass in dieser Formel die Reste R, R₁, R₂ und R₃ miteinander identisch sind.

Die in den erfindungsgemässen Kunststoffzusammensetzungen als Weichmacher verwendeten Benzhydrol-tetracarbonsäuretetraester werden vorzugsweise durch eine Reduktion der entsprechenden Benzophenon-tetracarbonsäuretetraester hergestellt. Die Reduktion der Benzophenon-tetracarbonsäuretetraester wird vorzugsweise mit einem Metallhydrid durchgeführt, beispielsweise mit Natriumborhydrid. Diese Reduktion wird im allgemeinen in einem geeigneten Lösungsmittel durchgeführt, beispielsweise einem Alkohol.

Zur Herstellung der bevorzugten Benzhydrol-tetracarbonsäuretetraester der Formel I wird als Ausgangsmaterial der Benzophenon-tetracarbonsäuretetraester der folgenden Formel II 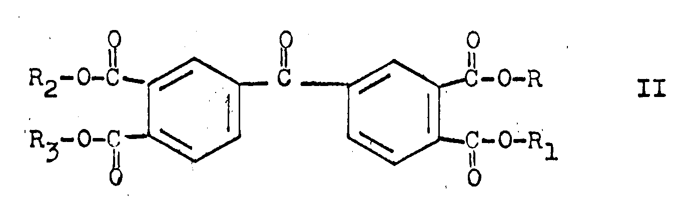
verwendet, wobei in diesem Ausgangsmaterial die Reste R, R₁, R₂ und R₃ die gleiche Bedeutung besitzen wie in Formel I.

Die Kunststoffkomponente der erfindungsgemäss weichgemachten Kunststoffzusammensetzungen kann aus einer grossen Anzahl an Kunststoffen ausgewählt werden. Vorzugsweise ist jedoch diese Kunststoffkomponente ein relativ hydrophober Kunststoff.

Als Beispiele für verwendbare Kunststoffe seien genannt:
Polyäthylen, Polypropylen, Polyvinylhalogenide, Polystyrol, Polyester, Polyamide, Polymethacrylnitril, Polyacrylnitril, Polyurethane, Polycarbonate, Polyvinylidenhalogenide, relativ hydrophobe Zellulosederivate oder Copolymerisate aus zwei oder mehr Monomereinheiten. In entsprechenden Copolymerisaten können gegebenenfalls auch geringere Anteile an stärker hydrophilen Monomerkomponenten enthalten sein, wie zum Beispiel Monomereinheiten, die als Substituenten Hydroxylgruppen, niedere aliphatische Aether oder Estergruppierungen und gegebenenfalls sogar Carboxylgruppen in geringen Anteilen tragen. Als Beispiele für derartige Monomereinheiten mit stärker hydrophilen Eigenschaften seien diejenigen genannt, die von Vinylalkohol, Acrylsäure, Methacrylsäure oder Vinylacetat stammen.

In den Monomereinheiten der Polymermaterialien können ferner irgendwelche hydrophobe Substituenten enthalten sein, wie zum Beispiel Alkylgruppen, wenn beispielsweise Vinyltoluol als Monomermaterial oder Monomerkomponente eingesetzt wird.

Die Polymermaterialien können ferner auch noch ungesättigte Gruppierungen aufweisen, und beispielsweise in der Polymerkette Alkingruppen oder Alkengruppen tragen, wie dies der Fall ist, wenn ein Dien, beispielsweise Butadien, als Monomerkomponente eingesetzt wird.

Als Vinylhalogenide, bzw. Vinylidenhalogenide können in den Copolymerisaten, bzw. Homopolymerisaten entsprechende Bromide oder Fluoride eingesetzt werden, bevorzugt sind Jedoch die Chloride.

Speziell vorteilhaft erwiesen sich die erfindungsgemässen Benzhydrol-tetracarbonsäuretetraester als Weichmacher für Vinylchloridhomopolymerisate oder Vinylchloridcopolymerisate.

Damit eine entsprechende Weichmachung der Kunststoffe erreicht wird, ist in den erfindungsgemässen Kunststoffzusammensetzungen vorzugsweise pro 10 Gew.-Teilen an dem Kunststoff, mindestens ein Gew.-Teil an dem Benzhydrol-tetracarbonsäure-tetraester-Weichmacher enthalten.

Speziell bevorzugt sind jedoch Kunststoffzusammensetzungen, die eine hohe Elastizität und eine gute Haftfähigkeit aufweisen. In diesem Falle enthalten die eine ionenselektive Komponente enthaltenden Kunststoffzusammensetzungen wesentlich höhere Mengen an dem Benzhydrol-tetracarbonsäuretetraester, der dann in den entsprechenden Kunststoffzusammensetzungen als elastifizierende und Haftfähigkeit verleihende Komponente wirkt.

Wenn in den erfindungsgemässen Kunststoffzusammensetzungen die Kunststoffkomponente ein Vinylchloridhomopolymerisat oder -copolymerisat ist, dann erhält man entsprechende Zusammensetzungen mit hoher Haftfähigkeit und hoher Elastizität, indem man pro 10 Gew.-Teile des Kunststoffes 15-80 Gew.-Teile, insbesonders 20-70 Gew.-Teile an dem Benzhydrol-tetracarbonsäuretetraester einsetzt. Als spezielles Beispiel sei eine Mischung genannt, die die ionenselektive Komponente enthält und in der pro 30 Gew.-Teilen an Polyvinylchlorid 70 Gew.-Teile an dem Benzhydroltetracarbonsäuretetraester enthalten sind.

Die Obergrenze an dem als elastifizierender Zusatz wirkenden Benzhydrol-tetracarbonsäuretetraester ist dadurch gegeben, dass die entsprechenden, eine ionenselektive Komponente enthaltenden Kunststoffzusammensetzungen, beispielsweise Kunststoff-Formkörper in Form von Folien oder Kunststoff-Formkörper, die sich als fest haftende Schicht auf einem entsprechenden Substrat befinden, bei der Temperatur, wo die entsprechenden Produkte verwendet werden sollen, nicht fliessen dürfen. Im allgemeinen ist eine Verwendung der Kunststoffe bei Zimmertemperatur vorgesehen und deshalb dürfen die entsprechenden Kunststoffe bei Zimmertemperaturen oder Temperaturen knapp oberhalb derselben, beispielsweise 40° C, nicht fliessen. Wenn der entsprechende Kunststoff Polyvinylchlorid ist, dann dürfen pro 10 Gew.-Teile dieses Kunststoffes nicht mehr als 90 Gew.-Teile an dem Benzhydrol-tetracarbonsäuretetraester beigegeben werden. Die Obergrenze an dem elastifizierenden Benzhydrol-tetracarbonsäuretetraester, die nicht überschritten werden soll, variiert Jedoch mit dem eingesetzten Kunststoff und kann in manchen Fällen schon bei 70 bis 80 Gew.-Teilen des Tetraesters pro 10 Gew.-Teilen des Kunststoffes liegen.

Kunststoffe, die durch die Beigabe der oben genannten grossen Mengen an dem Benzhydrol-tetracarbonsäuretetraesters haftfähig und elastisch gemacht wurden, können durch einfaches Aufpressen an einem Substrat befestigt werden.

Kunststoff-Formkörper, die unter Verwendung der Benzhydrol-tetracarbonsäuretetraester weichgemacht wurden, können beispielsweise in Form eines Blockes, eines Stabes, eines Rohres, eines Fadens, eines Filamentes, eines Stranges, einer Folie oder eines Blattes vorliegen. Wenn die entsprechenden Kunststoff-Formkörper pro 10 Gew.-Teile an dem Kunststoff 9 - 90 Gew.-Teile, vorzugsweise 12 - 85 Gew.-Teile, an dem Benzhydrol-tetracarbonsäuretetraester-Weichmacher enthalten, dann sind sie elastisch und haftfähig.

Folienförmige oder blattförmige Kunststoff-Formkörper, beispielsweise entsprechende, hohe Gehalte an dem Tetracarbonsäuretetraester aufweisende elastische und haftfähige Folien oder Blätter können durch Vergiessen einer Lösung aus dem Kunststoff und dem Benzhydrol-tetracarbonsäuretetraester in einem geeigneten Lösungsmittel hergestellt werden. Beispielsweise kann eine die ionenselektive Komponente enthaltende Lösung aus 10 Gew.-Teilen Polyvinylchlorid und 30 Gew.-Teilen Benzhydrol-tetracarbonsäuretetraester in einer ausreichenden Menge an Lösungsmittel, beispielsweise Tetrahydrofuran, unter Anwendung von üblichen Vergiesstechniken vergossen werden und man kann so folienförmige Kunststoff-Formkörper herstellen, die Dicken im Bereich von 3 »m bis 200 »m aufweisen. Derartige Folien sind sehr klebrig, haben eine hohe Elastizität und eine hohe Haftfähigkeit auf verschiedenen Substraten, wie zum Beispiel Silizium enthaltenden Substraten oder Kunststoffmaterialien, beispielsweise Poly(methylmethacrylat) oder Polyvinylchlorid.

Entsprechende Formkörper in Form von Blättern oder Folien besitzen eine hohe Elastizität und gleichzeitig vorteilhafte mechanische Eigenschaften, insbesondere eine hohe Reissfestigkeit. Derartige haftfähige elastische, folienförmige Kunststoff-Formkörper können in ungedehntem Zustand auf ein Substrat aufgebracht werden, wobei eine gute Haftfähigkeit erreicht wird. Ferner ist es möglich, entsprechende elastische folienförmige Kunststoff-Formkörper auf die Oberfläche eines Substrates in gedehntem Zustand aufzubringen, wobei angenommen werden kann, dass durch die elastischen Rückstellkräfte der gedehnten Folie die Haftfestigkeit auf dem Substrat noch mehr erhöht wird. In beiden Fällen erhält man jedenfalls auf dem Substrat eine dauerhafte Haftung der Folie.

Es ist ferner möglich, die Kunststoffe zusammen mit der ionenselektiven Komponente und mit dem Benzhydrol-tetracarbonsäuretetraester in einem Lösungsmittel zu lösen, vorzugsweise einem flüchtigen organischen Lösungsmittel, und diese Lösung auf ein Substrat aufzubringen, beispielsweise aufzustreichen, aufzusprühen oder aufzuschleudern, wobei sich dann nach dem Abdampfen des Lösungsmittels auf dem Substrat ein fest haftender Film des elastifizierten Kunststoffes ausbildet.

Kunststoffe, welche mit Hilfe der erwähnten grossen Mengen an dem Benzhydrol-tetracarbonsäuretetraester elastisch und haftfähig gemacht wurden, können beispielsweise auf die folgenden Substrate aufgebracht werden: Substrate aus Kunststoffen, beispielsweise Poly(methylmethacrylat) oder Poly(vinylchlorid), oder Silizium enthaltende Substrate. Bevorzugte Silizium enthaltende Substrate sind Glassubstrate, Quarzglassubstrate, Substrate aus silanisiertem Glas, Substrate aus Silikonen, Substrate aus elementarem Silizium, beispielsweise Substrate aus dotierten Silizium-Einkristallen, wobei diese Einkristalle im allgemeinen mit Siliziumoxid der Formel SiO₂ oder Siliziumnitrid der Formel Si₃N₄ beschichtet sind.

Auf all diesen Substraten weisen die bevorzugten erfindungsgemässen Kunststoffzusammensetzungen, die durch die erwähnten hohen Gehalte an dem Benzhydrol-tetracarbonsäure-tetraester elastifiziert und haftfähig gemacht wurden, eine sehr gute Haftung auf, und zwar unabhängig davon, ob beispielsweise entsprechende elastische und haftfähige Kunststoff-Folien an den entsprechenden Substraten durch Aufpresen befestigt werden oder ob auf den Substraten eine Schicht des entsprechenden weichgemachten Kunststoffes aufgebracht wird, indem man eine Lösung des elastifizierten Kunststoffes in einem relativ flüchtigen organischen Lösungsmittel aufträgt und dieses Lösungsmittel dann wieder abdampft.

Werden in den erfindungsgemässen Kunststoffzusammensetzungen pro 10 Gew.-Teile an dem Kunststoff nur etwa 1 - 9 Gew.-Teile an dem Benzhydroltetracarbonsäure-tetraester eingesetzt, dann wird dadurch eine Weichmachung erreicht, die entsprechenden Kunststoffe, beispielsweise Kunststoff-Formkörper in Form von Folien oder Bändern, sind jedoch nicht hochelastisch und sie weisen auch keine gute Haftfähigkeit auf verschiedenen Substraten auf.

Unabhängig davon, ob die erfindungsgemässen Kunststoffzusammensetzungen durch Verwendung geringerer Mengen an dem Benzhydrol-tetracarbonsäuretetraester weichgemacht wurden oder durch Verwendung der erwähnten hohen Mengen an dem Benzhydrol-tetracarbonsäure-tetraester elastifiziert und haftfähig gemacht wurden, besteht der wesentliche Vorteil der Benzhydrol-tetracarbonsäure-tetraester darin, dass sie in der Kunststoffmatrix verankert sind. Durch diese stark verminderte Wanderung oder Migration des Benzhydrol-tetracarbonsäuretetraesters in der Kunststoffmatrix werden Nachteile beseitigt, welche bei bisher allgemein verwendeten Weichmachern anzutreffen waren. Probleme bezüglich einer Migration des Weichmachers in der Kunststoffmatrix traten bei den bisher häufig verwendeten Weichmachern auf Basis von Estern, beispielsweise Estern von Dicarbonsäuren auf, wie zum Beispiel den entsprechenden Estern der Phthalsäure, der Sebacinsäure oder der Adipinsäure, und diese Nachteile waren auch bei den Weichmachern auf Basis von Estern der Phosphorsäure und Aetherweichmachern, wie zum Beispiel o-Nitrophenyloctyläther, festzustellen.

Durch die verminderte Wanderung oder Migration der Benzhydrol-tetracarbonsäuretetraester in der Kunststoffmatrix wird ausserdem verhindert, dass dieser Weichmacher von flüssigen Medien, die mit dem weichgemachten oder plastifizierten Kunststoff in Berührung stehen, ausgelaugt wird, beispielsweise durch verschiedene Lösungsmittelsysteme, wie zum Beispiel wässrige Lösungen. Ferner wird auch verhindert, dass der Weichmacher in Feststoffe einwandert, welche mit den erfindungsgemässen Kunststoffzusammensetzungen in inniger Berührung stehen, beispielsweise Schichten eines weiteren Materials, das auf erfindungsgemäss weichgemachte Kunststoff-Folien aufgebracht ist.

Die Auslaugung bisher verwendeter Weichmacher aus ionenselektiven Kunststoff -zusamenensetzungen führte vor allem in denjenigen Einsatzgebieten zu Problemen, wo die Kunststoffe mit eiweisshaltigen wässrigen Lösungen in Berührung standen. Durch entsprechende Versuche wurden die Vorteile der Benzhydrol-tetracarbonsäuretetraester im Vergleich zu bisher verwendeten Weichmachern bewiesen.

So konnte gezeigt werden, dass aus einer Membran auf Basis von 30 Gew.-Teilen Polyvinylchlorid plus 70 Gew.-Teilen des Benzhydroltetracarbonsäuretetraesters als elastifizierender Komponente dieser Bestandteil praktisch nicht ausgelaugt wird, wenn eine entsprechende Membran oder Folie mit einer Albuminlösung während mehrerer Tage in Kontakt gehalten wird. Im Gegensatz dazu wird bei Verwendung einer äquivalenten Menge eines Sebacinsäureesters als Weichmacher unter den gleichen Bedingungen fast 90 % des ursprünglich vorhandenen Weichmachers ausgelaugt. Bei diesem Test war der veresternde Alkohol des Benzhydrol-tetracarbonsäuretetraesters und auch der veresternde Alkohol des Sebacinsäureesters ein geradkettiges Alkanol mit 9 Kohlenstoffatomen.

Diese extrem geringe Auslaugbarkeit der Benzhydrol-tetracarbonsäuretetraester aus Kunststoffen ist von ausschlaggebender Bedeutung, wenn die entsprechenden Kunststoffe während ihrer Verwendung mit irgendwelchen flüssigen Materialien, beispielsweise wässrigen Lösungen, in Berührung stehen.

Wenn die erfindungsgemässen Kunststoffzusammensetzungen zusätzlich zu der ionenselektiven Komponente, der Kunststoffkomponente und dem Benzhydrol-tetracarbonsäuretetraester-Weichmacher noch eine weitere Komponente enthalten, deren Molekulargewicht nicht sehr klein ist, also beispielsweise eine Komponente, deren Molekulargewicht 100 beträgt oder höher liegt, dann wird auch die Wanderung oder Migration dieser weiteren Komponente in der Matrix des Kunststoffes durch den Benzhydroltetracarbonsäuretetraester vermindert oder verhindert.

In den entsprechenden Kunststoffzusammensetzungen wird dementsprechend auch ein Eluieren dieser weiteren Komponente durch Lösungsmittel, mit welchen das Kunststoffmaterial in Berührung steht, verhindert, und es wird ferner auch verhindert, dass diese weitere Komponente in einen anderen Feststoff einwandert, der mit dem erfindungsgemässen Kunststoffmaterial in inniger Berührung steht.

Die Vorteile, die weiter oben im Zusammenhang mit der Verhinderung der Migration des Weichmachers in der Kunststoffmatrix erläutert wurden, gelten also auch für zusätzliche Komponenten, sofern deren Molekulargewicht nicht ziemlich gering ist.

Es zeigte sich ferner, dass der in den erfindungsgemässen Kunststoffzusammensetzungen enthaltene Benzhydrol-tetracarbonsäuretetraester auch das Einwandern von Fremdmaterialien in den Kunststoff verhindert, sofern diese Fremdmaterialien kein besonders geringes Molekulargewicht aufweisen, also beispielsweise ein Molekulargewicht von 100 oder höher. Dementsprechend wird durch den Benzhydrol-tetracarbonsäuretetraester auch eine Einwanderung von Fremdkomponenten eines höheren Molekulargewichts in das Kunststoffmaterial durch Lösungen verhindert, die mit dem Kunststoffmaterial in Berührung stehen und das gleiche trifft auch für Feststoffe zu, die in innigem Kontakt mit dem erfindungsgemäss weichgemachten Kunststoff stehen.

Die in den erfindungsgemässen Kunststoffzusammensetzungen enthaltenen ionenselektiven Komponenten weisen Molekulargewichte auf, die über 100 liegen. Dementsprechend werden diese ionenselektiven Komponenten durch den Benzhydrol-tetracarbonsäuretetraester in der Kunststoffzusammensetzung verankert und dadurch wird ein Eluieren der ionenselektiven Komponente verhindert, wenn die entsprechenden Zusammensetzungen mit einem Lösungsmittel in Berührung stehen.

Die erfindungsgemässen Kunststoffzusammensetzungen, die eine gegenüber Kationen oder Arionen selektive Komponente enthalten, können als ionenempfindliche Teile von entsprechenden Vorrichtungen zur Bestimmung der Konzentration der Kationen oder Anionen in vielen Bereichen eingesetzt werden, beispielsweise als ionenselektive Membranen. Im allgemeinen enthalten diese Kunststoffzusammensetzungen relativ hohe Anteile an Weichmachern.

In der Literatur sind eine grosse Anzahl an entsprechenden ionenselektiven Komponenten beschrieben, im allgemeinen handelt es sich dabei um entsprechende kationselektive oder anionselektive Dicarbonsäurediamide. Es sei in diesem Zusammenhang auf die USA-Patentschrift 2 957 607 verwiesen, wo entsprechende kationselektive Komponenten genannt sind.

In ionenselektiven Membranen werden die Weichmacher im allgemeinen in solchen Mengen eingesetzt, dass die ionenselektive Komponente vollständig gelöst in der Weichmacherkomponente vorliegt. Typischerweise enthalten derartige ionenselektive Teile 30 - 40 Gew.-% an dem Kunststoff, 60 - 70 Gew.-% an der Weichmacherkomponente und 0,1 - 5 Gew.-% an der ionenselektiven Komponente.

Wie bereits weiter oben erläutert wurde, werden aus erfindungsgemässen Kunststoffzusammensetzungen, die Benzhydrol-tetracarbonsäuretetraester-Weichmacher auch dann praktisch nicht ausgelaugt, wenn sie in sehr hohen Mengen als elastifizierende und haftfähig machende Komponente eingesetzt werden. Diese Vorteile sind von ausschlaggebender Bedeutung, wenn die erfindungsgemässen Kunststoffzusammensetzungen ionenempfindliche Teile zur Bestimmung der Konzentration von Ionen in Testlösungen sind. Von besonderem Vorteil ist in diesem Zusammenhang ausserdem, dass die ein Molekulargewicht von mehr als 100 aufweisenden ionenempfindlichen Komponenten durch den Benzhydrol-tetracarbonsäuretetraester ebenfalls in der Kunststoffmatrix verankert werden.

Entsprechende ionenempfindliche Teile, beispielsweise ionenempfindliche Membranen, kommen bei ihrer Verwendung mit den Lösungen in Berührung, in denen die Ionenkonzentrationen bestimmt werden sollen, beispielsweise mit wässrigen Lösungen. Entsprechende bisher bekannte ionenempfindliche Teile hatten oft nur eine relativ kurze Lebensdauer, weil die Weichmacherkomponente von den Probelösungen aus den entsprechenden Teilen eluiert wurde. Insbesondere hatten derartige ionenempfindliche Membranen dann eine kurze Lebensdauer, wenn sie mit eiweissenthaltenden wässrigen Lösungen in Berührung kamen, wie dies der Fall ist, wenn sie im klinischen Bereich zur Bestimmung der Ionenkonzentrationen in Körperflüssigkeiten, beispielsweise Blut, eingesetzt werden.

Ionenempfindliche Teile, welche erfindungsgemäss einen Benzhydrol-tetracarbonsäuretetraester als Weichmacher enthalten, weisen die erwähnten Nachteile von bisher bekannten ionenempfindlichen Teilen, die andere, bisher zu diesem Zweck eingesetzte Weichmacher enthielten, nicht auf. Entsprechende erfindungsgemässe ionenselektive Membranen weisen im Vergleich zu bisher bekannten ionenselektiven Membranen, deren Weichmacher beispielsweise einer der erwähnten Ester von Dicarbonsäuren war, eine stark verlängerte Lebensdauer auf.

Bevorzugte erfindungsgemässe ionenempfindliche Membranen, welche die genannten Benzhydrol-tetracarbonsäuretetraester als Weichmacher enthalten, sind solche, deren Kunststoffkomponente Polyvinylchlorid ist. Vorzugsweise enthalten diese ionenempfindlichen Membranen 10 Gew.-Teile an dem Polyvinylchlorid, 15 - 90 Gew.-Teile, vorzugsweise 20 - 70 Gew.-Teile, an dem Benzhydrol-tetracarbonsäuretretraester und ferner 0,1 - 1 Gew.-Teile an der ionenselektiven Komponente zur Bestimmung der vorgesehenen Anionen oder Kationen.

Wie bereits weiter vorne klargelegt wurde, sind die Weichmacher, die in derartigen ionenempfindlichen Membranen bisher eingesetzt wurden, meist bestimmte Aether, sowie ferner Ester von Dicarbonsäuren oder Ester von Phosphorsäuren. Es wurde nun völlig unerwartet gefunden, dass die erfindungsgemässen Benzhydrol-tetracarbonsäuretetraester zu vielen weiteren unerwarteten Vorteilen führen, wenn sie als Weichmacher in ionenselektiven Membranen eingesetzt werden, und zwar im Vergleich zu Weichmachern, die bisher zu diesen Zwecken eingesetzt wurden.

In den Benzhydrol-tetracarbonsäuretetraestern, die als Weichmacher in den erfindungsgemässen Kunststoffzusammensetzungen eingesetzt werden, muss jede der vier Alkoholgruppen, wie bereits erwähnt wurde, von einem Alkanol, Alkenol oder Alkinol mit jeweils 4 - 24 Kohlenstoffatomen stammen. In bevorzugten Benzhydrol-tetracarbonsäuretetraestern weisen die entsprechenden Alkoholkomponenten 6 - 20 Kohlenstoffatome, und speziell bevorzugt, 9 - 19 Kohlenstoffatome auf. Die entsprechenden Alkohole sind ferner vorzugsweise geradkettige Alkohole oder die entsprechenden Alkohole weisen eine aliphatische Hauptkette mit höchstens drei Verzweigungsstellen auf. Derartige lange aliphatische Ketten verleihen den entsprechenden Benzzydrol-tetracarbonsäuretetraestern lipophile Eigenschaften. Ausserdem vergrössern diese langen aliphatschen Ketten die dynamische Viskosität der Benzhydroltetracarbonsäuretetraester aufgrund der grossen Molekülmasse dieser Substanzen und aufgrund von Wechselwirkungen zwischen den langen aliphatischen Ketten.

Diese langen aliphatischen Ketten führen ferner zu der bereits erwähnten stark verminderten Beweglichkeit der Benzhydrol-tetracarbonsäuretetraester in dem Polymergrundgerüst der Kunststoffkomponente, weil die langen aliphatischen Ketten der Ester sich zwischen die Polymerketten lagern und offensichtlich Verwicklungen zwischen den langen aliphatischen Ketten der Tetraester und den Polymerketten auftreten. Durch diese Verwicklungen wird offenbar auch die Beweglichkeit von weiteren Komponenten eines nicht zu geringen Molekulargewichts, also eines Molekulargewichts von mehr als 100, in derartigen System aus Polymergrundgerüst plus Benzhydrol-tetracarbonsäuretetraesterWeichmacher plus der erwähnten weiteren Komponente verringert.

Es zeigte sich, dass in entsprechenden Systemen, welche erfindungsgemäss Benzhydrol-tetracarbonsäuretetraester als Weichmacher enthalten, die Migration der ionenselektiven Komponente in dem weichgemachten Kunststoffmaterial drastisch verringert wird.

Entsprechende Tests wurden mit Membranen durchgeführt, die als ionenselektive Komponente das N,N'-Bis [(11-äthoxy-carbonyl)undecyl]N,N'-dimethyl-2,3-naphthalindioxydiacetamid enthalten, das in der Veröffentlichung von D. Amman, R. Bissig, M. Güggi, E. Pretsch, W. Simon, I.J. Borowitz und L. Weiss in Helv. Chim. Acta 58 (1975) 1535 beschrieben ist. In entsprechenden Membranen, in welchen dar Kunststoff Polyvinylchlorid ist und die ferner die angegebene ionenempfindliche Komponente enthalten, ist die Beweglichkeit dieser ionenempfindlichen Komponente drastisch vermindert, wenn erfindungsgemäss ein Benzhydrol-tetracarbonsäuretetraester als elastifizierende Komponente in entsprechenden ionenempfindlichen Membranen eingesetzt wird. Dies wurde im Vergleich zu entsprechenden Membranen zu Vergleichszwecken gezeigt, welche die gleiche ionenempfindliche Komponente in einer Polyvinylchloridpolymermatrix enthalten, jedoch in Kombination mit dem zum Stande der Technik gehörenden Weichmacher Sebacinsäure-2-äthyl-hexylester (Bis(2-äthyl hexyl)-sebacinat).

Ionenempfindliche Elektroden wurden mit Membranen ausgerüstet, welche jeweils einen Gew.-Teil der bereits genannten ionenselektiven Komponente, 70 Gew.-Teile entweder eines Benzhydrol-tetracarbonsäuretetraesters als Weichmacher oder 70 Gew.-Teile des zum Stande der Technik gehörenden Sebacinsäure-2-äthyl-hexylesters als Weichmacher und ausserdem 30 Gew.-Teile Polyvinylchlorid enthielten. Dabei zeigte es sich, dass die Lebensdauer der Elektroden, die mit den Membranen, enthaltend die Benzhydrol-tetracarbonsäuretetraester, ausgestattet waren, unter den gleichen Arbeitsbedingungen etwa das zehnfache betrug, verglichen mit den Elektroden, die mit den Membranen ausgestattet waren, welche den zum Stande der Technik gehörenden Weichmacher enthielten. Diese Vorteile zeigten sich auch, wenn in dem Benzhydrol-tetracarbonsäuretetraester der veresternde Alkohol identisch war mit der Alkoholkomponente Sebacinsäureester, also beispielsweise in beiden Fällen die entsprechenden Ester 2-Äthyl-hexylester waren.

Die durch die Benzhydrol-tetracarbonsäuretetraester sehr stark verminderte Mobilität der ionenselektiven Komponente in solchen ionenselektiven Membranen ist dort besonders vorteilhaft, wo ganz kleine, mehrere Ionen testende Systeme eingesetzt werden, weil in diesen miniaturisierten Systemen Bereiche, die für verschiedene Arten an Ionen empfindlich sind, in naher Umgebung angeordnet sind. Ein Nachteil derartiger Systeme ist derjenige, dass eine wechselweise Verunreinigung der einzelnen Bereiche mit den ionenempfindlichen Komponenten der benachbarten Bereiche auftreten kann, weil bei einem derartigen Sensor in der Membranbeschichtung die ionenempfindlichen Komponenten der einzelnen Bereiche ineinander diffundieren können. Dadurch, dass durch die Benzhydrol-tetracarbonsäuretetraester diese Beweglichkeit der ionenempfindlichen Komponente in den einzelnen Membranabschnitten vermindert wird, kann eine derartige wechselweise Verunreinigung praktisch vollständig verhindert werden.

Es wurde ferner festgestellt, dass trotz der Hemmung der Beweglichkeit und Diffusion von grossen Molekülen, wie zum Beispiel den ionenempfindlichen Komponenten, in ionenempfindlichen Membranen, welche die erfindungsgemässen Benzhydrol-tetracarbonsäuretetraester enthalten, die Diffusion von kleinen Molekülen in entsprechenden ionenselektiven Teilen, beispielsweise ionenselektiven Membranen, nicht behindert wird. So wird beispielsweise die Diffusion von Kohlendioxid, Sauerstoff und anderen Gasen in ionenselektiven Membranen nicht behindert, die als Weichmacher die Benzhydrol-tetracarbonsäuretetraester enthalten.

Aus diesem Grunde ist es möglich, unter Verwendung der Benzhydrol-tetracarbonsäuretetraester als elastifizierende Komponente, Membranen herzustellen, welche die Bestimmung von Kohlendioxid ermöglichen. Derartige Membranen sind in der Veröffentlichung von J.W. Ross, J.H. Riseman und J.A. Krüger in Pure Appl. Chem. 36 (1973) 473, mit dem Titel "Potentiometric gas sensing electrodes", beschrieben. Ferner können auch entsprechende Membranen für Sensoren hergestellt werden, die sowohl eine Ionenempfindlichkeit als auch eine hohe Gaspermeabilität erfordern. Beispielsweise werden solche Membransysteme zur Messung von Bicarbonat in der Veröffentlichung von U. Oesch, E. Malinowska und W. Simon mit dem Titel "Bicarbonate-selective electrode based on planar thin membrane technology", Anal. Chem. 59, Seite 2131, 1986, beschrieben. So diffundiert Kohlendioxid durch ionenempfindliche Membranen, welche die Benzhydrol-tetracarbonsäuretetraester als elastifizierende Komponente enthalten, rasch genug um eine Bestimmung dieses Anions in wässrigen Systemem zu ermöglichen. Das gleiche trifft auch für Sauerstoff und andere Gase zu und dementsprechend können die entsprechenden Membranen zusätzlich zu der Polymermatrix und Benzhydroltetracarbonsäuretetraestern als elastifizierender Komponente noch eine entsprechende ionenempfindliche Komponente enthalten, welche die Bestimmung des Gases ermöglicht, beispielsweise die Bestimmung von Sauerstoff in Körperflüssigkeiten, wie zum Beispiel Blutserum.

Ein weiterer Vorteil von ionenempfindlichen Membranen, welche durch die Verwendung der angeführten hohen Mengen an einem Benzhydrol-tetracarbonsäuretetraester elastisch und haftfähig gemacht wurden, besteht darin, dass derartige Membranen auf einen Elektrodenkörper, beispielsweise aus Glas oder silanisiertem Glas, aufgebracht werden können, und dass sie dann an dem Elektrodenkörper ausreichend fest haften, so dass sie durch ein Reiben mit dem Finger nicht davon entfernt werden. Wenn bei der Verwendung derartiger Elektroden zur Bestimmung der Konzentration entsprechender Kationen diese Membranen mit einem flüssigen Medium in Berührung gebracht werden, dann lösen sich die entsprechenden Kunststoff-Folien von dem Elektrodenkörper nicht ab.

Die Herstellung von Benzhydrol-tetracarbonsäuretetraestern wird anhand der Beispiele 1 und 2 erläutert und die nachfolgenden Beispiele 3 - 6 betreffen erfindungsgemässe Kunststoffzusammensetzungen.

### Beispiel 1

### Herstellung eines Benzhydrol-tetracarbonsäuretetraesters

Anhand dieses Beispiels wird die Herstellung des Benzhydrol-tetracarbonsäuretetraesters der Formel I 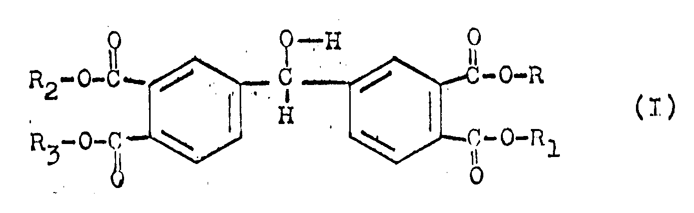
erläutert, wobei in dieser Formel jeder der Reste R, R₁, R₂ und R₃ die Bedeutung eines geradkettigen Alkylrestes mit 11 Kohlenstoffatomen aufweist.

### A) Herstellung des entsprechenden Benzophenon-tetracarbonsäuretetraesters

Zunächst wird der Benzophenon-tetracarbonsäuretetraesters der Formel II 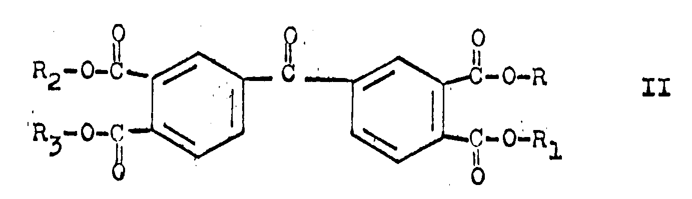
hergestellt, in dem ebenfalls die Reste R, R₁, R₂ und R₃ sämtlich n-Undekanreste sind.

Bei diesem Herstellungsverfahren setzte man zu einer Lösung, die 5 g (0,0155 Mol) an 3, 3', 4, 4'-Benzophenon-tetracarbonsäure-dianhydrid und 10,96 g (0,0636 Mol) an 1-Hydroxyundekan in 100 ml Toluol enthielt, 1 g Schwefelsäure, gelöst in 5 ml an Toluol unter Rühren bei Zimmertemperatur langsam zu. Nachdem die gesamte Menge an Schwefelsäure zugesetzt worden war, wurde die Mischung vier Stunden lang unter Rückfluss gekocht und das bei der Veresterungsreaktion abgespaltete Wasser wurde dabei aus der Rückflusskolonne abgetrennt.

Anschliessend wurde das Lösungsmittel abgedampft, der Rückstand in Chloroform gelöst und die organische Phase mit Wasser und zweimal mit einer 10 %-igen wässrigen Natriumbicarbonatlösung gewaschen.

Nach dem Abdampfen des Chloroforms erhielt man 13,8 g (entsprechend 91 % der Theorie) an dem rohen Benzophenon-tetracarbonsäure-tetraester. Dieser wurde durch Flashchromatographie (35 k Pa) auf Silikagel 60 (Teilchengrösse entsprechend einer lichten Maschenweite von 230 - 400 mesh ASTM) gereinigt, wobei als Laufmittel eine Mischung aus einem Volumenteil Essigsäureäthylester und vier Volumenteilen Hexan verwendet wurde.

Die Elementaranalyse ergab für das erhaltene Produkt der Summenformel C₆₁H₉₈O₉ (Molgewicht 974,45) die folgenden Werte:

| | | |
|---|---|---|
| berechnet: | C = 75,11 | H = 10,13 |
| gefunden: | C = 75,12 | H = 10,13. |

Das Infrarotspektrum, aufgenommen in Tetrachlorkohlenstoff zeigte Peaks bei 1730 (Aryl-COO, st.) und 1670 (Aryl-CO-aryl, st.).

Die Viskosität betrug 902,5 cP bei 22° C.

### B) Herstellung des Benzhydrol-tetracarbonsäuretetraesters

95 mg (2,5 mmol) an Natriumborhydrid wurden in 10 ml Methanol gelöst und diese Lösung wurde tropfenweise zu einer gerührten Lösung von 2 g (2,05 mmol) des gemäss Abschnitt (A) hergestellten Benzophenon-tetracarbonsäuretetraesters, gelöst in 50 ml Methanol zugesetzt. Die Umsetzung wurde in einer Stickstoffatmosphäre bei Zimmertemperatur durchgeführt. Nachdem 2 ½ Stunden lang gerührt worden war, setzte man 10 ml einer 3-molaren Essigsäure zu und rührte eine Stunde weiter. Anschliessend wurde das Lösungsmittel abgedampft und der Rückstand in 100 ml einer 0,1 molaren wässrigen Natriumkarbonatlöung aufgelöst und mit Chloroform extrahiert.

Nach dem Abdampfen des Chloroformes erhielt man 1,97 g des entsprechenden rohen Benzhydrol-tetracarbonsäuretetraesters, entsprechend 98 % der theoretischen Ausbeute.

Dieses rohe Produkt wurde durch Flashchromatographie (35 k Pa) auf Silicagel 60 (Teilchengrösse entsprechend einer lichten Maschenweite von 230 - 400 mesh ASTM) gereinigt, wobei als Laufmittel eine Mischung aus einem Volumenteil Essigsäureäthylester und vier Volumenteilen Hexan verwendet wurde.

Der erhaltene reine Benzhydrol-tetracarbonsäuretetraester wies einen Schmelzpunkt von 28° C auf.

Die Elementaranalyse dieses Produktes der Summenformel C₆₁H₁₀₀O₉ ergab die folgenden Werte:

| | | |
|---|---|---|
| berechnet: | C 74,96 % | H 10,31 % |
| gefunden: | C 74,88 % | H 10,43 %. |

Das Infrarotspektrum, aufgenommen in (CDCl₃), das Massenspektrum und die kernmagnetischen Resonanzspektren, jeweils aufgenommen in (CDCl₃), und zwar sowohl das ¹H-NMR (300 MHz), als auch das ¹³C-NMR (25 MHz),
bestätigten die angenommene Struktur.

### Beispiel 2

### A) Herstellung des Benzophenon-carbonsäuretetraesters

Nach dem in Beispiel 1, Abschnitt (A), beschriebenen Verfahren wurde ein Benzophenon-tetracarbonsäuretetraester hergestellt, der ebenfalls unter die dort genannte Formel II fällt, wobei jedoch jetzt jeder der Reste R, R₁, R₂ und R₃ ein geradkettiger Alkylrest mit 6 Kohlenstoffatomen war.

Die Herstellung erfolgte in der gleichen Weise wie in Beispiel 1, Abschnitt (A), beschrieben, doch wurde anstelle des 1-Hydroxyundekanes jetzt eine äquivalente Menge an dem 1-Hydroxyhexan verwendet.

### B) Herstellung des Benzhydrol-tetracarbonsäuretetraesters

Nach dem in Beispiel 1, Abschnitt (B), beschriebenen Verfahren wurde der gemäss Abschnitt (A) erhaltene Bensophenon-tetracarbonsäuretetraester unter Bildung des entsprechenden Benzhydrol-tetracarbonsäuretetraesters reduziert.

### Beispiel 3

Anhand dieses Beispiels wurde die Auswaschbarkeit der erfindungsgemässen Benzhydrol-tetracarbonsäuretetraester aus Kunststoffen im Vergleich zu bisher bekannten Weichmachern getestet. Es wurden jeweils Folien hergestellt, die aus 70 Gew.-Teilen des erfindungsgemässen Weichmachers bzw. eines bisher zu dem angegebenen Zweck eingesetzten Weichmachers, und aus 30 Gew.-Teilen Polyvinylchlorid zusammengesetzt waren. Das eingesetzte Polyvinylchlorid war ein hochmolekulares Polyvinylchlorid, das von der Firma Fluka erhältlich ist. Es wurden Folien hergestellt, welche die folgenden Weichmacher enthielten:

### Folie I

Diese Folie enthielt den nach dem Verfahren gemäss Beispiel 1 hergestellten Benzhydrol-tetracarbonsäuretetraundecylester und dieser wird in der Folge als BHT-11 abgekürzt.

Diese Folie wurde mit entsprechenden Folien verglichen, welche die gleiche Menge an Weichmachern zu Vergleichszwecken enthielten. Dieses waren die Folien II und III.

### Folie II

Diese enthielt als Weichmacher den Sebacinsäuredioctylester (Dioctyl-Sebacat), abgekürzt als DOS.

### Folie III

Diese enthielt als Weichmacher den ortho-Nitrophenyl-octyläther, abgekürzt als o-NPOE.

Die Folien zu Vergleichszwecken II und III enthielten Weichmacher, die häufig als Weichmacher in ionenempfindlichen Membranen eingesetzt werden, also solchen, die zusätzlich noch eine ionenempfindliche Komponente enthalten. Im vorliegenden Fall wurden aber entsprechende Folien ohne Gehalt an einer ionenempfindlichen Komponente getestet, weil lediglich die Auswaschbarkeit des Weichmachers durch wässrige Medien getestet werden sollte.

Die Folien I, II und III wurden in eine wässrige Albuminlösung eingebracht, die 70 g Albumin pro Liter enthielt. Dabei wurde jeweils eine solche Menge an Albuminlösung verwendet, dass pro Volumenteil Folie 1000 Volumenteile der Albuminlösung vorhanden waren. Die in der Albuminlösung befindlichen Folien hatten jeweils eine Dicke von 100 »m und sie wurden in der Lösung fünf Tage lang bei Zimmertemperatur auf einer Schüttelmaschine geschüttelt.

Nach dieser Behandlung wurden die Folien mit Wasser gewaschen und dann auf ihren Gehalt an Weichmachern, bezogen auf die ursprüngliche Weichmachermenge, getestet.

Die Menge an Weichmacher, die jede der Folien vor der Behandlung mit der Albuminlösung enthielt (also der Weichmachergehalt von 70 Gew.-%, bezogen auf das Gesamtgewicht der Folie), wurde mit 100 % festgesetzt.

Es zeigte sich dabei, dass in der Folie I nach diesem Test noch mindestens 99,5 % der ursprünglichen Weichmachermenge nach dieser Behandlung vorhanden waren.

Im Gegensatz dazu enthielt die Folie zu Vergleichszwecken II, welche unter die Verwendung des bekannten Weichmachers DOS hergestellt wurde, nurmehr 70,5 % der ursprünglichen Weichmachermenge, während die Folie zu Vergleichszwecken III, welche unter Verwendung des bekannten Weichmachers o-NPOE, hergestellt wurde, nurmehr 43,9 % des ursprünglich vorhandenen Weichmachers enthielt.

### Beispiel 4

In diesem Beispiel wurde die Migration eines erfindungsgemässen Weichmachers in einer Folie mit der Migration eines bisher bekannten Weichmachers verglichen.

Die in diesem Beispiel getesteten Folien waren die Folie I des Beispiels 3, welche 70 Gew.-% des Benzhydrol-tetracarbonsäure-tetraundecylesters und 30 Gew.-% Polyvinylchlorid enthielt, und ferner die Folie zu Vergleichszwecken II, welche 70 Gew.-% des Sebacinsäuredioctylesters und 30 Gew.-% Polyvinylchlorid enthielt.

Der Test wurde durchgeführt, indem man diese Folien einer Dicke von 100 »m mit wesentlich dickeren Polyvinylchloridschläuchen in Berührung brachte.

Als Polyvinylchloridschlauch wurde in diesem Test ein solcher verwendet, wie er im Handel erhältlich ist, und zwar ein entsprechender Schlauch, der wenn auf ihn eine ionenempfindliche Membran aufgebracht ist, als katheterartige Elektrode zur Bestimmung von Ionen, beispielsweise im Blut herangezogen werden kann. Entsprechende Polyvinylchloridschläuche enthalten Weichmacher und ein wesentlicher Nachteil bisher bekannter derartiger katheterartiger Elektroden bestand darin, dass ihre Lebensdauer sehr begrenzt war, weil der Weichmacher aus dem Polyvinylchloridschlauch in die ionenempfindliche Membran einwanderte und andererseits der Weichmacher der ionenempfindlichen Membran in den Polyvinylchloridschlauch einwanderte.

Mit diesem Test sollte überprüft werden, ob durch Verwendung der Benzhydroltetracarbonsäuretetraester als Weichmacher in entsprechenden Membranen die oben erwähnten Migrationsprobleme der jeweiligen Weichmacher vermieden werden können.

Die bei diesem Test verwendeten im Handel erhältlichen Polyvinylchloridschläuche enthielten 36,6 Gew.%, bezogen auf das Gesamtgewicht des Schlauches an dem Weichmacher Diäthylhexylphthalat, abgekürzt als DEHP, und 73,4 Gew.-%, bezogen auf das Gesamtgewicht des Schlauches, an Polyvinylchlorid.

Bei diesem Test wurden die Folien I, bzw. II auf den Polyvinylchloridschlauch aufgelegt und darauf festgepresst und es wurde nach einer bestimmten Kontaktzeit festgestellt, wie stark die Wanderung des Weichmachers aus der Folie in den Polyvinylchloridschlauch, bzw. des Weichmachers des Polyvinylchloridschlauches in die Folie war.

Bei diesem Test zeigte sich, dass bei Verwendung der erfindungsgemässen Folie I nach einer Kontaktzeit mit dem Polyvinylchloridschlauch von 9480 Minuten nur eine äusserst geringfügige Migration des erfindungsgemässen Weichmachers BHT-11 der Folien I in den Polyvinylchloridschlauch und ausserdem auch nur eine äusserst geringfügige Migration des Weichmachers DEHP des Polyvinylchloridschlauches in die Folie I stattgefunden hatte.

Im Gegensatz dazu konnte bei den Folien zu Vergleichszwecken II bereits nach einer Kontaktzeit von 237 Minuten eine deutliche Migration des Weichmachers DOS der Folie in den Polyvinylchloridschlauch und auch des Weichmachers DEHP des Polyvinylchloridschlauches in die Folie II zu Vergleichszwecken festgestellt werden.

### Beispiel 5

Anhand dieses Beispiels soll gezeigt werden, dass Polyvinylchloridfolien, welche die bevorzugte Menge von mindestens 12 Gew.-Teilen, und speziell bevorzugt, mindestens 15 Gew.-Teilen, des Benzhydrol-tetracarbonsäuretetraesters pro 10 Gew.-Teile eines Polyvinylchloridkunststoffes enthalten, eine hervorragende Haftfähigkeit auf unterschiedlichen Substraten aufweisen, während entsprechende Polyvinylchloridfolien, welche einen erfindungsgemässen Benzhydrol-tetracarbonsäuretetraester in Mengen von weniger als 12 Gew.-Teilen pro 10 Gew.-Teile Kunststoff enthalten, nicht diese hervorragenden Hafteigenschaften auf verschiedenen Substraten aufweisen.

Alle in diesem Beispiel miteinander verglichenen Polyvinylchloridmembranen sind jedoch Membranen, welche den nach dem Verfahren gemäss Beispiel 1 hergestellten Benzhydrol-tetracarbonsäuretetraundecylester als Weichmacher, bzw. als haftfähig machende und elastifizierende Komponente enthalten.

Zur Herstellung der Membranen wurde eine entsprechende Menge des Polyvinylchlorides und des Benzohydrol-tetracarbonsäuretetraesters in einer ausreichenden Menge an Tetrahydrofuran gelöst und diese Lösung wurde zu Membranen einer Dicke von etwa 100 »m vergossen.

Die eingesetzten Mengen an Polyvinylchlorid und dem erfindungsgemässen Benzhydrol-tetracarbonsäuretetraester sind in Gew.-% in der nachfolgenden Tabelle angeführt.

| Membran | Gew.-Teile Polyvinylchlorid | Gew.-Teile Benzhydrol-tetracarbonsäure-tetraester |
|---|---|---|
| A | 10 | 90 |
| B | 30 | 70 |
| C | 40 | 60 |
| D | 54 | 46 |

Jede der Folien A, B, C und D wurde bezüglich ihrer Haftfestigkeit auf Substraten aus Glas, Quarzglas und Plexiglas getestet. Auf die entsprechenden Substrate wurde jeweils die zu testende Folie in leicht gestrecktem Zustand aufgebracht.

Sämtliche Folien blieben auf den drei Substraten nach der Aufbringung haften. Wenn man jedoch anschliessend über die Stelle, wo die Folien aufgebracht waren, mit dem Finger rieb, dann wurden durch diese Behandlung die Folien A, B und C nicht abgeschält, jedoch löste sich unter diesen Bedingungen die Folie D.

Mit dem Messer konnten alle Folien A, B, C und D von sämtlichen Substraten wieder abgekratzt werden.

Die erfindungsgemässe Folie D enthielt pro 10 Gew.-Teilen an Polyvinylchlorid weniger als 12 Gew.-Teile des Benzhydrol-tetracarbonsäuretetraesters, so dass ihr Gehalt an diesem Weichmacher unterhalb des besonders bevorzugten Bereiches liegt. Dieser geringere Gehalt an Weichmacher bewirkte, dass die fragliche Folie nicht mehr die speziell erwünschte extrem hohe Haftfähigkeit auf unterschiedlichen Substraten aufweist.

Von den hier getesteten Folien zeigte die Folie B die besten Resultate bezüglich der Reissfestigkeit.

### Beispiel 6

Kunststoffe, bei denen eine hohe Elastizität und hohe Haftfähigkeit nicht erwünscht ist, enthalten geringere Anteile an den erfindungsgemässen Benzhydroltetracarbonsäure-tetraestern, beispielsweise 1 - 7 Gew.-Teile pro 10 Gew.-Teile Kunststoff. Sie werden dort verwendet, wo eine gute Verankerung des Weichmachers in der Kunststoffmatrix von ausschlaggebender Bedeutung ist.

## Patentansprüche

1. Kunststoff, der einen Tetracarbonsäure-tetraester-Weichmacher enthält, dadurch gekennzeichnet, dass der Weichmacher ein Benzhydrol-tetracarbonsäuretetraester ist, in welchem die Carboxylatgruppen des Tetraesters direkt an die aromatischen Kerne des Benzhydroles gebunden sind und wobei die veresternden Alkoholkomponenten dieses Tetraester-Weichmachers Alkanole, Alkenole oder Alkinole mit 4 - 24 Kohlenstoffatomen sind und wobei gegebenenfalls in den aromatischen Ringen des Benzhydrol-Grundgerüstes und/oder in den Resten der Alkoholkomponente nicht-ionische Substituenten vorhanden sind, und dass der Kunststoff als weiteren Bestandteil eine ionenselektive Komponente enthält.

2. Kunststoff gemäss Anspruch 1, dadurch gekennzeichnet, dass er eine gegenüber Anionen empfindliche ionenselektive Komponente oder eine gegenüber Kationen empfindliche ionenselektive Komponente enthält.

3. Kunststoff gemäss Anspruch 1 oder 2, dadurch gekennzeichnet, dass der Weichmacher ein Benzhydroltetracarbonsäuretetraester ist, in welchem die Alkoholkomponente des Tetraesters ein geradkettiger Alkohol oder ein höchstens drei Verzweigungsstellen aufweisender Alkohol ist.

4. Kunststoff nach einem der Ansprüche 1 bis 3, dadurch gekennzeichnet, dass in dem Benzhydroltetracarbonsäuretetraester jeder der beiden Benzolringe zwei Estergruppierungen trägt, wobei in den beiden Benzolkernen die Anordnung der Estergruppierungen vorzugsweise identisch ist, bezogen auf das Kohlenstoffatom der Benzolkerne, welches an die Gruppierung 〉CH-OH des Benzhydroles gebunden ist.

5. Kunststoff nach Anspruch 4, dadurch gekennzeichnet, dass der Benzhydrol-tetracarbonsäuretetraester die folgende Formel I 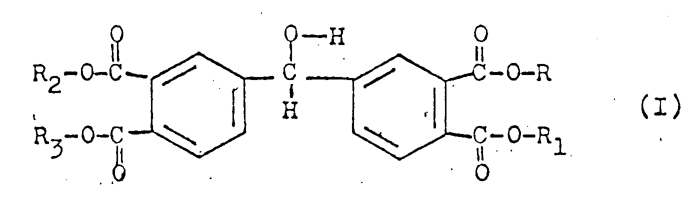 aufweist, wobei jeder der Reste R, R₁, R₂ und R₃ ein Alkylrest, Alkenylrest oder Alkinylrest mit 4 - 22 Kohlenstoffatomen, vorzugsweise 6 - 20 Kohlenstoffatomen, ist und wobei vorzugsweise die Reste R, R₁, R₂, bzw. R₃ geradkettig sind oder höchstens drei Verzweigungsstellen aufweisen.

6. Kunststoff nach einem der Ansprüche 1 - 5, dadurch gekennzeichnet, dass in dem Tetraester-Weichmacher die vier Estergruppierungen die gleiche Struktur besitzen.

7. Kunststoff nach einem der Ansprüche 1 - 6, dadurch gekennzeichnet, dass der den Tetraester-Weichmacher enthaltende Kunststoff ein Polyäthylen, ein Polypropylen, ein Polyvinylhalogenid, ein Polystyrol, ein Polyester, ein Polyamid, ein Polyacrylnitril, ein Polyurethan, ein Polycarbonat, ein Polyvinylidenhalogenid oder ein Copolymerisat aus zwei oder mehr Monomereinheiten ist, wobei in Copolymerisaten gegebenenfalls auch geringere Anteile an stärker hydrophilen Monomerkomponenten, beispielsweise Vinylalkohol, enthalten sein können.

8. Kunststoff gemäss Anspruch 7, dadurch gekennzeichnet, dass der Kunststoff in Form einer ionenempfindlichen Membran vorliegt, wobei diese ionenempfindliche Membran
10 Gew.-Teile an Polyvinylchlorid,
15 - 90 Gew.-Teile, vorzugsweise 20 - 70 Gew.-Teile an dem Tetracarbonsäuretetraester-Weichmacher und
0,1 - 1 Gew.-Teile an der ionenselektiven Komponente enthält.

9. Kunststoff gemäss Anspruch 1 mit hoher Elastizität und Haftfähigkeit, dadurch gekennzeichnet, dass er pro 10 Gew.-Teile des Kunststoffes 12 - 90 Gew.-Teile, vorzugsweise 15 - 80 Gew.-Teile, und speziell bevorzugt 20 - 70 Gew.-Teile des Weichmachers enthält.

10. Kunststoff nach Anspruch 9, dadurch gekennzeichnet, dass der Benzhydrol-tetracarbonsäuretetraester der Formel I 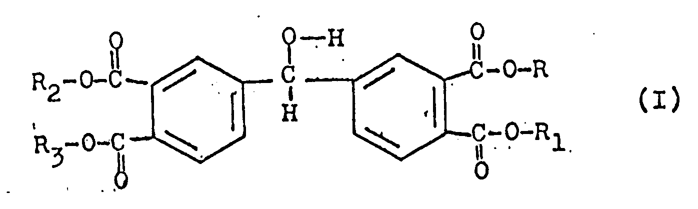 entspricht, wobei jeder der Reste R, R₁, R₂ und R₃ ein Alkylrest, Alkenylrest oder Alkinylrest mit 4 - 22 Kohlenstoffatomen, vorzugsweise 6 - 20 Kohlenstoffatomen, ist und wobei vorzugsweise die Reste R, R₁, R₂, bzw. R₃ geradkettig sind oder höchstens drei Verzweigungsstellen aufweisen.

11. Kunststoff nach Anspruch 9 oder 10, dadurch gekennzeichnet, dass in dem Tetraester-Weichmacher die vier Estergruppierungen die gleiche Struktur besitzen.

12. Kunststoff nach einem der Ansprüche 9 bis 11, dadurch gekennzeichnet, dass der Kunststoff ein Polyäthylen, ein Polypropylen, ein Polyvinylhalogenid, ein Polystyrol, ein Polyester, ein Polyamid, ein Polyacrylnitril, ein Polyurethan, ein Polycarbonat, ein Polyvinylidenhalogenid oder ein Copolymerisat aus zwei oder mehr Monomereinheiten ist, wobei in Copolymerisaten gegebenenfalls auch geringere Anteile an stärker hydrophilen Monomerkomponenten, beispielsweise Vinylalkohol, enthalten sein können.

13. Kunststoff nach einem der Ansprüche 9 bis 12, dadurch gekennzeichnet, dass er ein Kunststoff-Formkörper ist, beispielsweise ein Formkörper, der in Form eines Fadens, eines Stranges, eines Filamentes oder einer Folie vorliegt, wobei sich der Kunststoff-Formkörper gegebenenfalls in Berührung mit einem anderen Feststoff oder flüssigen Medium befindet, beispielsweise als haftender Film oder haftende Beschichtung auf einem festen Substrat oder der als folienförmiger Schichtträger für mindestens eine Schicht eines weiteren Materials dient.

## Claims

1. Synthetic polymer containing a tetracarboxylic acid tetraester plasticizer, **characterised in that** the plasticizer is a benzhydrol-tetracarboxylic acid tetraester in which the carboxylate groups of the tetraester are bonded directly to the aromatic nuclei of the benzhydrol and wherein the esterifying alcohol components of this tetraester plasticizer are alkanols, alkenols or alkinols with 4 - 24 carbon atoms and wherein optionally in the aromatic rings of the benzhydrol backbone and/or in the residues of the alcohol component non-ionic substituents are present, and that the synthetic polymer contains as a further constituent an ion-selective component.

2. Synthetic polymer according to claim 1, **characterised in that** it contains an ion-selective component sensitive with respect to anions or an ion-selective component sensitive with respect to cations.

3. Synthetic polymer according to claim 1 or 2, **characterised in that** the plasticizer is a benzhydrol-tetracarboxylic acid tetraester in which the alcohol component of the tetraester is a straight-chain alcohol or an alcohol possessing at most three branch points.

4. Synthetic polymer according to any one of claims 1 to 3, **characterised in that** in the benzhydrol-tetracarboxylic acid tetraester each of the two benzene rings bears two ester groups, wherein in the two benzene nuclei the arrangement of the ester groups is preferably identical in relation to the carbon atom of the benzene nuclei which is bonded to the 〉CH-OH group of the benzhydrol.

5. Synthetic polymer according to claim 4, **characterised in that** the benzhydrol-tetracarboxylic acid tetraester has the following Formula I 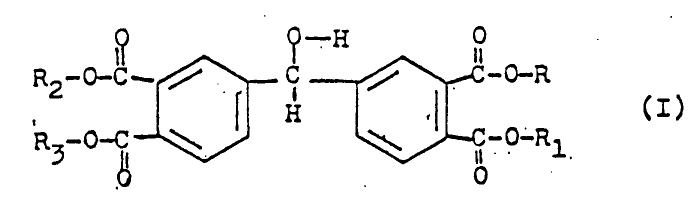 where each of the residues R, R₁, R₂ and R₃ is an alkyl residue, alkenyl residue or alkinyl residue with 4 - 22 carbon atoms, preferably 6 - 20 carbon atoms, and where preferably the residues R, R₁, R₂ and R₃ are straight-chain or possess at most three branch points.

6. Synthetic polymer according to any one of claims 1 - 5, **characterised in that** in the tetraester plasticizer the four ester groups possess the same structure.

7. Synthetic polymer according to any one of claims 1 to 6, **characterised in that** the synthetic polymer containing the tetraester plasticizer is a polyethylene, a polypropylene, a polyvinyl halide, a polystyrene, a polyester, a polyamide, a polyacrylonitrile, a polyurethane, a polycarbonate, a polyvinylidene halide or a copolymer consisting of two or more monomer units, wherein in copolymers optionally also smaller amounts of more strongly hydrophilic monomer components, for example vinyl alcohol, can be included.

8. Synthetic polymer according to claim 7, **characterised in that** the synthetic polymer is present in the form of an ion-sensitive membrane, wherein this ion-sensitive membrane contains
10 parts by wt of polyvinyl chloride,
15 - 90 parts by wt, preferably 20 - 70 parts by wt, of the tetracarboxylic acid tetraester plasticizer and
0.1 - 1 part by wt of the ion-selective component.

9. Synthetic polymer according to claim 1 with high elasticity and adhesiveness, **characterised in that** it contains for every 10 parts by wt of the synthetic polymer 12 - 90 parts by wt, preferably 15 - 80 parts by wt, and particularly preferably 20 - 70 parts by wt of the plasticizer.

10. Synthetic polymer according to claim 9, **characterised in that** the benzhydrol-tetracarboxylic acid tetraester corresponds to Formula I 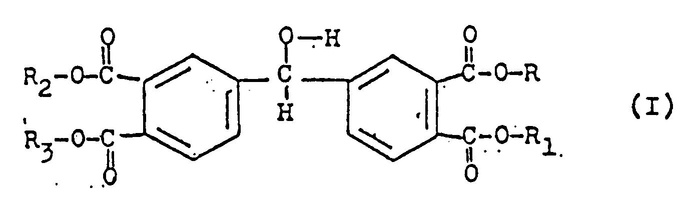 where each of the residues R, R₁, R₂ and R₃ is an alkyl residue, alkenyl residue or alkinyl residue with 4 - 22 carbon atoms, preferably 6 - 20 carbon atoms, and where preferably the residues R, R₁, R₂ and R₃ are straight-chain or possess at most three branch points.

11. Synthetic polymer according to claim 9 or 10, **characterised in that** in the tetraester plasticizer the four ester groups possess the same structure.

12. Synthetic polymer according to any one of claims 9 to 11, **characterised in that** the synthetic polymer is a polyethylene, a polypropylene, a polyvinyl halide, a polystyrene, a polyester, a polyamide, a polyacrylonitrile, a polyurethane, a polycarbonate, a polyvinylidene halide or a copolymer consisting of two or more monomer units, wherein in copolymers optionally also smaller amounts of more strongly hydrophilic monomer components, for example vinyl alcohol, can be included.

13. Synthetic polymer according to any one of claims 9 to 12, **characterised in that** it is a synthetic polymer moulding, for example a moulding which is present in the form of a thread, a strand, a filament or a film, wherein the synthetic polymer moulding is optionally in contact with another solid or liquid medium, for example as an adhering film or adhering coating on a solid substrate, or which serves as a film-type layer support for at least one layer of a further material.

## Revendications

1. Polymère synthétique, qui contient un plastifiant tétraester d'acide tétracarboxylique, caractérisé en ce que le plastifiant est un benzhydrole-tétraester d'acide tétracarboxylique, dont les groupes de carboxylate du tétraester sont directement attachés aux noyaux aromatiques du benzhydrole et dont les composantes d'alcools formant les esters de ce plastifiant tétraester sont des alcanoles, des alcènoles ou des alcynoles avec 4-24 atomes de carbone et dont, le cas échéant, il y a des groupes non-ioniques dans les noyaux aromatiques de la charpente du benzhydrole et/ou dans les restes de la composante d'alcool et que le polymère synthétique contient, comme composante supplémentaire, une composante iono-sélective.

2. Polymère synthétique suivant la revendication 1, caractérisé en ce qu'il contient une composante iono-sélective sensible aux anions ou une composante iono-sélective sensible aux cations.

3. Polymère synthétique suivant la revendication 1 ou 2, caractérisé en ce que le plastifiant est un benzhydrole-tétraester d'acide tétracarboxylique, dans lequel la composante d'alcool du tétraester est un alcool avec une chaîne droite et, a au maximum trois endroits de ramification.

4. Polymère synthétique suivant une des revendications 1 à 3, caractérisé en ce que chacun des deux noyaux de benzène dans le benzhydrole-tétraester d'acide tétracarboxylique porte deux groupes d'ester, dont, dans les deux noyaux de benzène, l'arrangement des groupes d'ester est par préférence identique, par rapport à l'atome de carbone des noyaux de benzène attaché au groupe 〉CH-OH du benzhydrole.

5. Polymère synthétique suivant la revendication 4, caractérisé en ce que le benzhydrole-tétraester d'acide tétracarboxylique présente la formule I suivante, 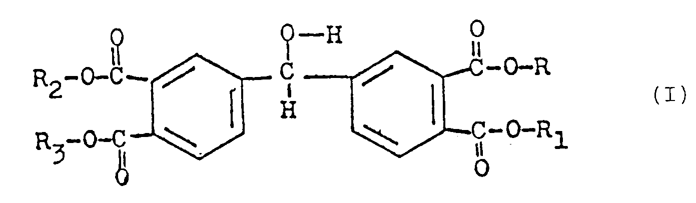 dont chacun des restes R, R₁, R₂ et R₃ est un reste d'alkyle, d'alcènyle ou d'alcyinyle avec 4 - 22 atomes de carbone, par préférence 6 - 20 atomes de carbone et dont, par préférence, les restes R, R₂, R₂ ou R₃ ont une chaîne droite ou bien avec, au maximum, trois endroits de ramification.

6. Polymère synthétique suivant une des revendications 1 - 5, caractérisé en ce que dans le plastifiant tétraester, les quatre groupes d'ester possèdent la même structure.

7. Polymère synthétique suivant une des revendications 1-6, caractérisé en ce que le polymère synthétique contenant le plastifiant tétraester est un polyéthylène un polypropylène, un halogénure de polyvinyle, un polystyrène, un polyester, un polyamide, un polyacrylnitrile, un polyuréthane, un polycarbonate, un halogénure de polyvinylidène ou un co-polymérisate de deux ou plusieurs unités de monomères, alors que dans des co-polymérisates il peut y avoir aussi, le cas échéant, des proportions faibles de composantes de monomère hydrophile plus fort, comme par exemple de l'alcool de vinyle.

8. Polymère synthétique suivant la revendication 7, caractérisé en ce que le polymère synthétique se présente sous forme d'une membrane iono-sensible, qui contient
10 parts en poids de chlorure de polyvinyle,
15 - 90 parts en poids, par préférence 20 - 70 parts en poids de tétraester d'acide tétracarboxylique comme plastifiant et
0,1 - 1 part en poids de la composante iono-sélective.

9. Polymère synthétique suivant la revendication 1, avec une élasticité et une adhésion élevées, caractérisé en ce qu'il contient sur 10 parts en poids du polymère synthétique 12 - 90 parts en poids, par préférence 15 - 80 parts en poids et particulièrement préféré 20-70 parts en poids du plastifiant.

10. Polymère synthétique suivant la revendication 9, caractérisé en ce que le benzhydrole-tétraester d'acide tétracarboxylique correspond à la formule I, 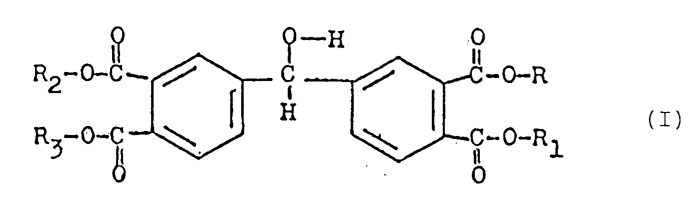 dont chacun des restes R, R₁, R₂ et R₃ est un reste d'alkyle, d'alcènyle ou d'alcynyle avec 4 - 22 atomes de carbone, par préférence 6 - 20 atomes de carbone et dont par préférence les restes R, R₂, R₂ ou R₃ ont une chaîne droite ou avec, au maximum, trois endroits de ramification.

11. Polymère synthétique suivant la revendication 9 ou 10, caractérisé en ce que, dans le plastifiant de tétraester, les quatre groupes d'ester possèdent la même structure.

12. Polymère synthétique suivant une des revendications 9 à 11, caractérisé en ce que le polymère synthétique est un polyéthylène, un polypropylène, un halogénure de polyvinyle, un polystyrène, un polyester, un polyamide, un polyacrylnitrile, un polyuréthane, un polycarbonate, un halogénure de polyvinylidène ou un copolymérisate de deux ou plusieurs unités de monomères, alors que, dans des co-polymérisates, il peut y avoir aussi, le cas échéant, des proportions faibles de composantes de monomère hydrophile plus forts, comme par exemple de l'alcool de vinyle.

13. Polymère synthétique suivant une des revendications 9 à 12, caractérisé en ce qu'il est un objet en plastique, par exemple un objet en plastique, qui en forme d'un fil, d'une barre, d'un filament ou d'un film, alors que l'objet en plastique se trouve, le cas échéant, en contact avec un autre solide, ou un milieu liquide, par exemple comme un film adhésif ou comme une couche adhésive sur un substrat solide ou qui sert comme porteur de couche sous forme de film pour au moins une couche d'un autre matériel.
